Europäisches Patentamt

European Patent Office

Office européen des brevets

(19)

(11) Publication number : **0 072 947 B2**

(12) # NEW EUROPEAN PATENT SPECIFICATION

(45) Date of publication of the new patent
specification : **12.01.94 Bulletin 94/02**

(51) Int. Cl.⁵ : **A61K 37/54**

(21) Application number : **82107092.7**

(22) Date of filing : **05.08.82**

(54) **Agent for the prevention and treatment of liver diseases.**

The file contains technical information
submitted after the application was filed and
not included in this specification

(30) Priority : **10.08.81 JP 125002/81**

(43) Date of publication of application :
**02.03.83 Bulletin 83/09**

(45) Publication of the grant of the patent :
**04.06.86 Bulletin 86/23**

(45) Mention of the opposition decision :
**12.01.94 Bulletin 94/02**

(84) Designated Contracting States :
**AT BE CH DE FR GB IT LI LU NL SE**

(56) References cited :
**EP-A- 0 059 346**
**EP-A- 0 063 395**
**WO-A-81/01242**
**BE-A- 684 147**
**FR-A- 2 448 903**
**FR-M- 3 536**
**LU-A- 59 502**
**Remington's Pharmaceutical Sciences, p.
972-975 (1975)**
**"Enzymtherapie" von Max Wolf und Karl
Ransberger, Wilhelm Maudrich Verlag, Wien
1970.**
**Firmemblatt der Mucos Emul-
sionsgesellschaft mbH, März 1981**
**J.C.Sachdev, "Antifatty Liver Action of Pro-
teolytic Enzymes of Animal and Plant Origin in
Pancreatic Duct-Ligated Rats", erschienen in
Gastroenterology 27, 3: 353-7(1954)**
**H.Feinberg et al.: "Antifatty Liver Action of
Papain and Ficin in Insulin-Treated, Depan-
creatized Dogs". Proc. Soc. Exp. Biol., Med.,
vol. 17, 1967, Seiten 161 f.**
**I.Innerfield et al., "The Rat Plasminogen Sys-
tem, Intrahepatic Fibrinolysis and Carbon Tet-
rachloride Hepatotoxicity". Thrombos.
Diathes. haemorrh.17, 1967, Stuttgart. Seiten
447 ff.**
**"Clinical Effects of Pronase(KHP-1) on Active
Chronic Hepatitis". The Igaku to Yakugaku"
(Medicine and Pharmacology) Japan. 1987,
18(4) 1073-1086.**

(56) References cited :
**Efficacy of Pronase on Hepatic His-
topathological Patterns in Patients with
Chronic Active Hepatitis: Evaluation by double
blind study" in "Kan Tan Sui"(Liver, Gall and
Pancreas) Japan, 1987, 15(4), pp. 819-832.**

(73) Proprietor : **KAKEN PHARMACEUTICAL CO.,
LTD.**
**No. 28-8, 2-chome, Honkomagome Bunkyo-Ku
Tokyo 113 (JP)**
Proprietor : **Fujisaki, Shigemi**
**1-48, Tonoyama-cho
Nishinomiya-shi Hyogo-ken (JP)**

(72) Inventor : **Fujisaki, Shigemi**
**1-48, Tonoyama-cho Nishinomiya-shi
Hyogo-ken (JP)**
Inventor : **Mitani, Mitsuaki**
**B-6-502, Kamitsuruma 1941
Sagimahara-shi Kanagawa (JP)**

(74) Representative : **VOSSIUS & PARTNER**
**Postfach 86 07 67
D-81634 München (DE)**

Jouve, 18, rue Saint-Denis, 75001 PARIS

## Description

Diseases of the liver are already well-known; however, in recent years in particular, viral hepatitis and alcoholic hepatitis, have shown a tendency to increase, posing a social problem. As a recent new theory propounds, there have been increasing cases of transitions from acute to chronic hepatitis, from chronic hepatitis to hepatocirrhosis, and from hepatochirrhosis to liver cancer. Therefore it is the pressing need of the hour and supremely important for saving human lives as well to cure these disorders of internal organs at the time of inflammation in order to prevent their transition to cancer.

Pharmaceutical compositions containing proteolytic and/or polysaccharide-mucopolysaccharide lysing enzymes as effective ingredients are disclosed in FR-A-2 448 903, BE-A-684 147, EP-A-59 346, EP-A-65 395, WO 81/01242 and Remington's Pharmaceutical Sciences, p.972-975. These known pharmaceutical compositions however are not provided for use in treating or preventing liver diseases.

It is the object of the present invention to provide a compound for the preparation of a pharmaceutical composition for preventing and treating chronic hepatitis. This object is achieved by providing the enzyme Pronase® for said use.

The subject matter of the present invention therefore is the use of the proteolytic enzyme pronase® for the preparation of a pharmaceutical composition for preventing and treating chronic hepatitis.

Another subject matter of the invention is a pharmaceutical composition for preventing and treating chronic hepatitis containing pronase® in an amount of 10,000-500,000 tyrosine units in an enteric preparation.

Pronase® employed in the present invention is a commercial product. Further it may be obtained by cultivating *Streptomyces griseus* followed by extracting and purifying the resulting culture broth.

Pronase® employed in the present invention can be administered orally as, for example, tablets, capsules, powders, granules, that are prepared by ordinary techniques of pharmaceutical manufacture or parenterally as, for example, injections, suppositories, ointments, that are prepared in a similar manner. The agent can also be used in combination with other pharmaceuticals, for example, other medicines for the liver, antibiotics, immunoactivators, antineoplastic agents and so forth. The dosage for attaining the objective, varying with the severity of disorder, method of administration, dosage forms, is 10-50,000 mg daily for an adult in the case nf oral administration and 10-1000 mg given as an enteric preparation. The oral dosage attaining the objective is 10,000-500,000 tyrosine units.

Pronase® employed in the present invention has the special feature that it is transmigrated in very large amounts to lesions of the liver. Tissues of the lesions show subsidence of edema, infiltration of round cells, production of new blood vessels, dissolution of fibrin, and an increase in polysaccharide-acidic ones in particular.-and curing reactions are observed to take place and be promoted in a chronic inflammation picture of the liver, that is, retroplasia or abnormal proliferation.

Example of a pharmaceutical preparation 1

To a mixture of 20 g of pronase® and 40 g of lactose is added 10 g of hydroxypropylmethylcellulose. The resulting mixture is made into granules, which are uniformly coated with cellulose acetate phthalate to prepare enteric granules.

Example of a pharmaceutical preparation 2

The pronase® enteric granules obtained in Example 1 are filled into capsules to prepare capsule preparations.

Clinical Example 1

Male, 58 years old

Diagnosis:

Hepatocirrhosis, chronic hepatitis.

Chief complaint:

Anorexia, mild pressure sensation at the right hypochondriac region, general fatigue.

2

Present illness:

The patient has had the foregoing symptoms for 2-3 years and was diagnosed as having hepatocirrhosis on May 24 at another hospital.

Present condition:

No abnormalities are observed except mild hypertrophy of the liver. Serum examinations and ultrasonography were made.

Ultrasonography:

No abnormalities in either kidney; mild hypertrophy of the spleen: the gallbladder exhibits large cysts and contains no gallstone; the choledochus has no dilatation; the pancreas exhibits mild swelling and the surface thereof is smooth; no significant changes are observed inside the pancreas.

Liver:

The surface is almost smooth but the tissue has mild atrophy; the liver lumen has become narrowed and curved; there are no localized abnormal pictures inside the liver; biopsy of liver cells revealed pictures of mild hepatocirrhosis and chronic hepatitis.

Examinations on liver functions:

As Table 1 shows, examinations and observations were made 5 times during a period of about 3 months extending from May 25 to July 30. The first medical examination revealed abnormalities in 5 items out of 13-an icterus index, GOT, GPT, LAP and $\gamma$-GTP.

Treatment with pronase®:

Empynase® (trade name; manufactured by Kaken Chemical Co., Ltd.; enteric preparation; one tablet contains 9,000 tyrosine units of pronase®) was internally administered in a dosage of 6 tablets daily, 2 tablets each after meals, starting on May 25. Examinations were made on June 5. Mild exacerbation was seen in every item in addition to the observation of an abnormal TTT value.

Sudden improvements were seen on June 18, only $\gamma$-GTP showing abnormalities. On June 10, $\gamma$-GTP also decreased. On July 30, all showed normal values.

Subjective symptoms improved, too, and the results of various examinations became normal. Thereafter, the internal administration was continued for a further 3 months to prevent a recurrence.

3

TABLE 1
Course of results of examinations on liver functions

| Items examined | Normal value | Day of examination | | | | |
|---|---|---|---|---|---|---|
| | | 5/25 | 6/5 | 6/18 | 7/10 | 7/30 |
| | | day 0 | day 10 | day 23 | day 45 | day 65 |
| Icterus index | 4—6 | *7 | *7 | 4 | 5 | 5 |
| TTT | 0.4—4.0 | 4 | *8 | 1.5 | 1.9 | 1.9 |
| ZTT | 4.0—12.0 | 8.6 | 10.5 | 7.4 | 7.6 | 6.7 |
| GOT | 8—40 | *55 | *95 | 33 | 26 | 20 |
| GPT | 5—35 | *36 | *64 | 26 | 27 | 20 |
| AL-P | 2.0—10.0 | 5.1 | 6.7 | 4.7 | 5.1 | 4.2 |
| LDH | 50—450 | 380 | 342 | 317 | 322 | 308 |
| LAP | 70—200 | *288 | *310 | 190 | 166 | 145 |
| γ-GTP | —40 | *284 | *251 | *139 | *59 | 38 |
| Total cholesterol | 130—230 | 191 | 218 | 186 | 188 | 175 |
| Total proteins | 6.5—8.3 | 7.6 | 7.5 | 6.8 | 7.5 | 7.1 |
| Albumin | 56—68 | 59.2 | 61.3 | 61.7 | 62.6 | 63.3 |
| A/G | 1.27—2.12 | 1.45 | 1.58 | 1.61 | 1.61 | 1.73 |

*: abnormal value.

Clinical example 2

Female; born in 1915

Diagnosis:

Chronic hepatitis.

Chief complaint:

General fatigue, anorexia.

Present illness:

For about 6 months the patient has complained of general fatigue and anorexia and lost her vigor to work.

Present condition:

The patient's face is somewhat pale with a shade of yellow. The patient has an enlarged abdominal part, which has no abnormalities such as retention of ascites.

Ultrasonography and roentgenography:

The kidneys on both sides and the spleen have no abnormalities. The gallbladder has mild hypertrophy but contains no gallstone. There are no significant changes in the pancreas, the large intestine, the small intestine, the stomach, or the duodenum.

Liver:

The surface is smooth. The liver lumen has become somewhat narrowed but no atrophy or localized abnormal pictures are observed in the liver tissue.

Examination on liver functions:

As Table 2 shows, examinations that had been carried out on November 7 revealed no abnormalities in 6 items among 13.

TABLE 2
Course of results of examinations on liver functions

| Items examined | Day of examination | | | | |
| --- | --- | --- | --- | --- | --- |
| | 11/7 | 1/30 next year | 3/5 | 5/18 | 6/5 |
| | day 0 | day 85 | day 119 | day 193 | day 211 |
| Icterus index | 4 | 5 | 6 | 4 | 4 |
| TTT | *6.1 | *7.4 | *5.4 | *5.0 | 3.8 |
| ZTT | *13.9 | *12.8 | *12.6 | 7.8 | 7.5 |
| GOT | *59 | *45 | 36 | 31 | 31 |
| GPT | *47 | *39 | 28 | 25 | 24 |
| AL-P | 6.6 | 8.1 | 5.7 | 5.5 | 5.3 |
| LDH | 411 | 356 | 385 | 323 | 320 |
| LAP | 165 | 153 | 151 | 127 | 120 |
| γ-GTP | *54 | 38 | 34 | 20 | 20 |
| Total cholesterol | 209 | 187 | 210 | 207 | 208 |
| Total proteins | *8.6 | *8.4 | 8.0 | 8.1 | 8.1 |
| Albumin | 58.1 | 57.1 | 57.5 | *55.5 | 57 |
| A/G | 1.38 | 1.33 | 1.35 | *1.25 | 1.28 |

*: abnormal value.

Treatment with pronase®:

Empynase®P was internally administered in a dosage of 6 tablets daily, 2 tablets each after meals, and the subsequent course observed. By January 30 the next year, γ-GTP became normal but the others. though somewhat improving, did not become restored to normal. By March 5, all except TTT and ZTT became normal. On May 18, TTT, albumin, and A/G showed abnormalities, but albumin and A/G were low. By June 6, all had become normal. This is 211 days after the start of the treatment.

Clinical Examples 3-6:

Treatment of liver disorders with pronase®. Each case received internally Empynase®P in a dosage of 6

As Table 3 further shows, Empynase®P was internally administered to 4 cases of chronic hepatitis; as a result, very favorable results were obtained in these cases that had been considered difficult to treat.

TABLE 3

| Clinical case | Disease | Sex Age | Number of items examined of abnormal liver functions | | Days of administration | Effect |
|---|---|---|---|---|---|---|
| | | | Before treatment | After treatment | | |
| 3 | chronic hepatitis | female 67 | 6 | 0 | 213 | " |
| 4 | " | male 56 | 5 | 0 | 35 | " |
| 5 | " | male 57 | 7 | 3 | 24 | effective |
| 6 | " | male 57 | 3 | 1 | 59 | " |

tablets daily.

**Claims**

1. The use of the proteolytic enzyme pronase® for the preparation of a pharmaceutical composition for preventing and treating chronic hepatitis.

2. An embodiment according to claim 1 for the preparation of an enteric composition.

3. A pharmaceutical composition for preventing and treating chronic hepatitis containing pronase® in an amount of 10,000-500,000 tyrosine units in an enteric preparation.


**Patentansprüche**

1. Verwendung des proteolytischen Enzyms Pronase® zur Herstellung eines Arzneimittels zur Vorbeugung und Behandlung von chronischer Hepatitis.

2. Ausführung nach Anspruch 1 zur Herstellung einer Magensäure-resistenten Zusammensetzung.

3. Arzneimittel zur Vorbeugung und Behandlung von chronischer Hepatitis, enthaltend Pronase® in einer Menge von 10.000 bis 500.000 Tyrosin-Einheiten in einer Magensäure-resistenten Zubereitung.


**Revendications**

1. Utilisation de l'enzyme protéolytique pronase® pour la préparation d'une composition pharmaceutique destinée à la prévention et au traitement de l'hépatite chronique.

2. Mode de réalisation selon la revendication 1 pour la préparation d'une composition entérique.

3. Composition pharmaceutique pour la prévention et le traitement de l'hépatite chronique contenant de la pronase® dans une quantité de 10 000-500 000 unités de tyrosine dans une préparation entérique.